# EUROPÄISCHE PATENTANMELDUNG

(11) **EP 4 122 652 A1**
(43) Veröffentlichungstag der Anmeldung: **25.01.2023**
(21) Anmeldenummer: 22196535.3
(22) Anmeldetag: 21.09.2017
(51) Int. Cl.: B25J 9/00, A61F 5/01

(54) **VORRICHTUNG ZUM UNTERSTÜTZEN BEIDER ARME EINES BENUTZERS**

(30) Priorität: 06.06.2017 DE 102017112436
(62) Teilanmeldung aus: 17783733.3
(71) Anmelder: Ottobock SE & Co. KGaA, 37115 Duderstadt (DE)
(72) Erfinder: MIZERA, Oliver, 37115 Duderstadt (DE); KURZWEG, Annedore, 37115 Duderstadt (DE); FOX, Samantha, 37115 Duderstadt (DE); SCHIRRMEISTER, Benjamin, 37115 Duderstadt (DE); KEHNEN, Meike, 37115 Duderstadt (DE); MOSLER, Lüder, 37115 Duderstadt (DE); WAGNER, Sonja, 37115 Duderstadt (DE); VOLLBRECHT, Matthias, 37115 Duderstadt (DE)
(74) Vertreter: Gramm, Lins & Partner Patent- und Rechtsanwälte PartGmbB

(57) **Zusammenfassung**

Die Erfindung betrifft eine Vorrichtung zum Unterstützen zweier Arme (4) eines Benutzers (2), wobei die Vorrichtung zwei Armstützelemente (6) mit je einer Armschale (10) zum Anlegen an jeweils einen Arm (4), wenigstens einen passiven Aktuator (26), der eingerichtet ist, eine Kraft auf wenigstens eines der Armstützelemente (6) aufzubringen, und wenigstens ein Gegenlager (14) für die aufzubringende Kraft aufweist, das wenigstens ein Gegenlagerelement (16) und wenigstens zwei Kraftübertragungselemente (18) aufweist, die eingerichtet sind, eine Gegenkraft von jeweils einem der Armstützelemente auf das Gegenlagerelement (16) zu übertragen, wobei die Kraftübertragungselemente (18) relativ zu dem Gegenlagerelement (16) bewegbar, insbesondere um wenigstens eine Rotationsachse drehbar, an dem Gegenlagerelement (16) angeordnet sind, wobei die Stärke der aufzubringenden Kraft einstellbar ist.

## Beschreibung

Die Erfindung betrifft eine Vorrichtung zum Unterstützen zweier Arme eines Benutzers, wobei die Vorrichtung zwei Armstützelemente mit je einer Armschale zum Anlegen an jeweils einen Arm, wenigstens einen passiven Aktuator, der eingerichtet ist, eine Kraft auf wenigstens eines der Armstützelemente aufzubringen, und wenigstens ein Gegenlager für die aufzubringende Kraft aufweist, das wenigstens ein Gegenlagerelement und wenigstens zwei Kraftübertragungselemente aufweist, die eingerichtet sind, eine Gegenkraft von jeweils einem der Armstützelemente auf das Gegenlagerelement zu übertragen.

Eine derartige Vorrichtung ist beispielsweise aus der US 2016/0081871 A1 bekannt. Sie verfügt über ein Gegenlagerelement, das in Form eines um den Rumpf des Benutzers herumlegbaren Gurtes ausgebildet ist. An ihm befinden sich zwei entlang des Rückens zur Schulter verlaufende Stützstreben, die oberhalb und seitlich neben den Schultern des Benutzers mit jeweils einem Gelenk verbunden sind, so dass der Arm angehoben werden kann. An den entsprechenden Gelenken sind Federelemente angeordnet, durch die eine nach oben gerichtete Kraft auf die Armschalen ausgeübt werden kann, so dass beispielsweise beim Heben schwerer Gegenstände oder beim Arbeiten über Kopf eine Unterstützung der Arme erfolgen kann. Sollen die Arme gesenkt werden, muss durch die Arme ein Druck auf die Armschalen ausgeübt werden, der die von den Federelementen aufgebrachte Kraft übersteigt, so dass die Arme absinken.

Aus der WO 2014/093408 A2 und der US 9,427,865 B2 ist eine ähnliche Vorrichtung bekannt, bei der als mechanischer Energiespeicher, der als passiver Aktuator wirkt, jeweils eine Feder, insbesondere eine Zugfeder vorgesehen ist, die mit einem Bowdenzug verbunden ist. Der Bowdenzug wird über eine Umlenkrolle geführt, so dass beim Verschwenken eines Armes, was eine Bewegung des Armstützelementes relativ zum Gegenlagerelement bedeutet, die Feder gedehnt wird, so dass der mechanische Energiespeicher mit Energie aufgeladen wird.

Insbesondere für den Fall, dass der Benutzer der Vorrichtung beispielsweise stolpert oder stürzt und sich beispielsweise abrollen muss, können derartige Vorrichtungen zu einer Gefahr für den Benutzer werden und zu Verletzungen führen. Dies geschieht, obwohl die außerhalb des Schultergelenks angeordneten Gelenke der Vorrichtung beispielsweise gemäß dem Stand der Technik möglichst exakt so angeordnet sind, dass ihre Gelenkachsen und Schwenkachsen durch die entsprechenden Achsen des Schultergelenks hindurchlaufen, so dass vermeintlich alle Bewegungen, die die natürliche Schulter und damit der Arm des Benutzers ausführen kann, nachgebildet werden können.

Eine aktive Vorrichtung, die Arme beim Arbeiten über Kopf unterstützt, ist aus der EP 3 156 193 A1 bekannt. Die Armschalen sind über eine Vielzahl verschiedener Gelenke und Verbindungsrahmenelemente miteinander verbunden. Dadurch sollen möglichst viele der Bewegungen, die ein Schultergelenk durchführen kann, auch mit der angelegten Vorrichtung möglich sein. Die Vorrichtung ist jedoch aufgrund der Vielzahl an Elementen groß, konstruktionsaufwendig und damit teuer. Hinzu kommt, dass die Vorrichtung das Schultergelenk durch zwei Scharniergelenke nachzubilden versucht. Dadurch sind nicht aller Bewegungen, die ein natürliches Schultergelenk ausführen kann, möglich. Insbesondere für das Anheben des Armes in einer bestimmten Richtung muss zunächst das dafür vorgesehene Gelenk in die richtige Position gedreht werden, damit seine Schwenkachse in die gewünschte Richtung zeigt. Dadurch müssen gegebenenfalls zusätzliche und unnatürliche Bewegungen ausgeführt werden, wodurch der Komfort und damit auch die Akzeptanz der Vorrichtung beim Benutzer reduziert wird.

Weitere Unterstützungsvorrichtungen, die insbesondere beim Heben von schweren Gegenständen oder beim Arbeiten über Kopf unterstützen, sind aus der WO 2014/195373 A1 und der US 2016/339583 A1 bekannt. Diese Vorrichtungen sind jedoch nur auf besondere Bewegungen, die es zu unterstützen gilt, ausgerichtet.

Der Erfindung liegt daher die Aufgabe zugrunde, diese Nachteile zu beheben oder zumindest zu mindern.

Die Erfindung löst die gestellte Aufgabe durch eine Vorrichtung gemäß dem Oberbegriff des Anspruchs 1, die sich dadurch auszeichnet, dass die Kraftübertragungselemente relativ zu dem Gegenlagerelement bewegbar, insbesondere um wenigstens eine Rotationsachse drehbar, an dem Gegenlagerelement angeordnet sind. Dazu können die Kraftübertragungselemente beispielsweise an dem Gegenlagerelement durch ein Scharnier, ein Kugelgelenk oder ein sonstiges Gelenk befestigt oder beispielsweise mit einem Ende des Kraftübertragungselementes in eine dafür vorgesehene Tasche oder Halterung am Gegenlagerelement eingesteckt oder eingeführt sein.

Der Erfindung liegt die Erkenntnis zugrunde, dass trotz der oftmals komplexen und aufwendigen Anordnung von Gelenken durch derartige Orthesen und Vorrichtungen im Bereich der Schulter des Trägers viele Bewegungen nicht nachgebildet werden können. Dies gilt insbesondere für die Bewegungen, bei denen sich die Orientierung und/oder Position des wenigstens einen Armstützelementes relativ zum Gegenlagerelement verändert und die nicht oder nicht nur durch eine Bewegung der Schulter, sondern beispielsweise durch Bewegungen des Rumpfes, der Wirbelsäule oder Schultergürtels hervorgerufen werden. Diese Bewegungen sind insbesondere in den geschilderten Situationen, in denen der Benutzer stolpert oder fällt, von größter Wichtigkeit, um den Sturz abzufangen und Verletzungen zu vermeiden.

Insbesondere liegt die Erkenntnis zugrunde, dass bei der aus der EP 3 156 193 A1 bekannten Vorrichtung die Scharniergelenke einen festen Bezugspunkt zu den anatomischen Gelenken benötigen und deshalb die Drehpunkte durch den Rahmen fest fixiert sind.

Durch die Bewegbarkeit der Kraftübertragungselemente relativ zu dem Gegenlagerelement wird in einer bevorzugten Ausgestaltung der Vorrichtung erreicht, dass die Bewegungsfreiheit wenigstens eines der Arme, bevorzugt beider Arme, durch die Vorrichtung nicht eingeschränkt wird.

Wird die Bewegungsfreiheit des Armes durch die Vorrichtung nicht eingeschränkt, wird darunter im Rahmen der vorliegenden Erfindung insbesondere verstanden, dass jede Bewegung, die der Benutzer der Vorrichtung ohne Vorrichtung durchführen kann, auch mit der Vorrichtung möglich ist, so dass er allenfalls minimal, also nicht nennenswert, eingeschränkt ist. Diese Bewegungen schließen insbesondere die Anteversion und Retroversion, also das Anheben des Arms nach vorn und hinten, die Abduktion und Adduktion, also das Anheben und Heranziehen des Armes in seitlicher Richtung und die Innenrotation und die Außenrotation im Schultergelenk ein. Insbesondere ist mit einer erfindungsgemäßen Vorrichtung eine Zirkumduktion in dem gleichen Grade möglich, wie sie der entsprechenden Person auch ohne die Vorrichtung möglich ist. Eine Zirkumduktion ist das Herumführen des Armes um die ausführbaren Hauptbewegungen der drei Gelenkachsen, insbesondere beim maximalen Bewegungsausschlag, bei dem sich ein unregelmäßiger Kegel ergibt, dessen Spitze im Schultergelenk liegt.

Vorzugsweise werden auch Bewegungen der Wirbelsäule, insbesondere eine Neigung der Wirbelsäule zur Seite und/oder nach vorn und hinten und/oder eine Verdrehung der Wirkbelsäule um ihre Längsachse, nicht durch die Vorrichtung behindert, eingeschränkt oder unmöglich gemacht. Vorzugsweise werden alle diese hier beschriebenen Bewegungen durch die Vorrichtung weder in ihrem maximalen Bewegungsausschlag, noch in einer Bewegungsreihenfolge eingeschränkt.

Die Armschale ist dabei bezüglich des wenigstens einen Gegenlagerelementes wenigstens in drei translatorischen und drei rotatorischen Freiheitsgraden bewegbar. Während Vorrichtungen aus dem Stand der Technik allenfalls rotatorische Freiheitsgrade erlauben, wird durch die erfindungsgemäße Vorrichtung erreicht, dass auch Bewegungen der Armschale relativ zum Gegenlagerelement ermöglicht sind, die beispielsweise lediglich eine translatorische Verschiebung entlang einer Raumrichtung betreffen. Dadurch kann die Armschale relativ zum Gegenlagerelement Bewegungen des Oberarms nachvollziehen und mit vollführen, die nicht allein aus Bewegungen der Schulter stammen, sondern beispielsweise eine Bewegung der Wirbelsäule oder eines anderen Körperteiles erfordern.

In einer bevorzugten Ausführungsform der Vorrichtung gemäß der hier beschriebenen Erfindung weist das Kraftübertragungselement ein Druckkraftübertragungselement, insbesondere eine Stange oder eine Schiene auf, an dem das Armstützelement um eine Schwenkachse schwenkbar angeordnet ist. Dabei ist es vielfach ausreichend, ein einziges Scharniergelenk mit einer Schwenkachse vorzusehen. Komplizierte Aufbauten mehrerer Gelenke, deren Schwenkachsen sich in einem Punkt, vorzugsweise virtuell im Schwenkpunkt des Kugelgelenks der natürlichen Schulter treffen, sind unnötig. Dadurch wird die Konstruktion stark vereinfacht. Zudem können die Herstellungskosten gesenkt werden.

Vorzugsweise befindet sich das Gelenk, durch das das Armstützelement mit dem Kraftübertragungselement verbunden ist, im angelegten Zustand der Vorrichtung im Bereich eines Schulterblattes, vorzugsweise am Schulterblatt des Benutzers. Dies bedeutet jedoch insbesondere nicht, dass das Gelenk im angelegten Zustand mit dem Körper des Benutzers in Kontakt ist, sondern lediglich, dass dieser Kontakt am Schulterblatt des Benutzers vorliegen würde, wenn das Gelenk an den Körper des Benutzers gedrückt werden würde.

Das Gegenlagerelement wird beim Betrieb der Vorrichtung an einem Gegenstand angelegt, auf den die wirkenden Kräfte übertragen werden sollen. Da der passive Aktuator eine Kraft auf das Armstützelement aufbringen soll, muss ein entsprechendes Gegenlager vorhanden sein, an dem er sich abstützen kann. Ein passiver Aktuator im Sinne der vorliegenden Erfindung ist insbesondere kein Motor. Die benötigte Energie, die er zum Aufbringen der Kraft benötigt, wird durch den Benutzer oder Träger der Vorrichtung aufgebracht. In der Regel wird die Kraft, die auf das Armstützelement aufgebracht werden soll, der Schwerkraft entgegenwirkend aufgebracht. Beim Anheben des Armes und des mit ihm verbundenen Armstützelementes wird folglich Energie aus einem Energiespeicher des passiven Aktuators entzogen. Bei der entgegengesetzten Bewegung, also dem Absenken des Armes, geschieht dies gegen die aufgebrachte Kraft. Es wird Energie dem Energiespeicher des passiven Aktuators zugeführt. Für einen passiven Aktuator im Sinne der vorliegenden Erfindung ist dies die einzige Energiequelle, wenn man von einer einstellbaren Vorspannung des Energiespeichers des passiven Aktuators absieht.

Vorteilhafterweise verfügt der passive Aktuator über wenigstens einen Energiespeicher, bevorzugt wenigstens einen mechanischen Energiespeicher. Dieser kann beispielsweise ein Federelement, ein Druckspeicher, ein pneumatisches und/oder hydraulisches System und/oder einen hydraulischen Energiespeicher aufweisen. Das Federelement kann beispielsweise direkt am Gelenk zwischen dem Druckkraftübertragungselement und dem Armstützelement in Form einer Rotationsfeder oder einer Konstantkraftfeder angeordnet sein. Auch elastische Elemente in Form von elastischen Seilen, beispielsweise Gummiseilen, sind vorstellbar, deren eines Ende an einem Teil des Armstützelementes angeordnet sind. Wird das Armstützelement relativ zum Druckkraftübertragungselement um die Schwenkachse herum verschwenkt, wird das elastische Element gedehnt oder gestaucht, so dass Energie dem mechanischen Energiespeicher zugeführt oder aus ihm abgezogen wird. Selbstverständlich sind auch andere Elemente, beispielsweise Gasdruckfedern oder Druckfedern, denkbar, für die eine Umlenkung verwendet wird, um aus der durch die Druckfeder bereitgestellten Druckkraft eine Zugkraft zu machen.

Der mechanische Energiespeicher kann an unterschiedlichsten Positionen der Vorrichtung angeordnet sein. Vorteilhafterweise wird eine Position gewählt, an der der für den Energiespeicher benötigte Bauraum vorhanden ist und der Energiespeicher selbst bei Bewegungen des Arms des Benutzers nicht stört. So kann er beispielsweise am Oberarm angeordnet sein.

In einer bevorzugten Ausgestaltung ist das Gegenlagerelement ein Anlageelement, insbesondere ein Gurt, ein Gürtel, eine Bandage oder ein Schalenelement, zum Anlegen an ein Körperteil, insbesondere einen Rumpf des Benutzers. Das Anlageelement ist vorzugsweise in ein Kleidungsstück, beispielsweise eine Hose integriert. Alternativ oder zusätzlich dazu weist das Gegenlagerelement wenigstens ein Schulterelement zum Anlegen an einer Schulter des Benutzers auf. Alternativ oder zusätzlich verfügt das Gegenlagerelement über wenigstens ein Bodenkontaktelement, so dass die Gegenkraft in den Boden einleitbar ist. Derartige Bodenkontaktelemente sind vorzugsweise als Exoskelette ausgebildet und aus dem Stand der Technik an sich bekannt. Es kann aktiv, beispielsweise motorgetrieben, oder passiv ausgebildet sein

Wird das Gegenlagerelement als Anlageelement für den Rumpf des Benutzers, insbesondere die Hüfte des Benutzers, ausgebildet, kann auf für den Benutzer besonders bequeme und komfortable Weise die benötigte Gegenkraft abgeleitet werden. Sie wird im Hüftbereich in den Körper des Benutzers eingeleitet und durch die Beine abgefangen. Die Verwendung eines Bodenkontaktelementes ist insbesondere dann von Vorteil, wenn die Vorrichtung verwendet wird, um beim Heben großer Lasten zu unterstützen. Würde in diesem Fall das Gegenlagerelement als Anlageelement für den Rumpf, insbesondere die Hüfte, des Benutzers ausgebildet, wäre die durch die schwere Last auftretende starke Zusatzbelastung zwar vom Schulterbereich des Benutzers abgeleitet worden, müsste jedoch durch die Beine weiterhin geleistet werden. Dem kann abgeholfen werden, indem zumindest auch ein Bodenkontaktelement verwendet wird. Dabei ist es möglich, zumindest einen Teil der Belastung, vorzugsweise jedoch die gesamte zusätzliche Belastung, in den Boden abzuleiten, so dass auch die Beine und insbesondere die Knie des Benutzers nicht überbelastet werden. Dazu können Orthesen, beispielweise aktive oder passive Exoskelette, wie sie aus dem Stand der Technik prinzipiell bekannt sind, verwendet werden, die sich von der Hüfte über das Knie und den Knöchel bis zum Fuß des Benutzers erstrecken.

Die Verwendung eines Schulterelementes zum Anlegen an die Schulter, das beispielsweise in Form von Rucksackträgern oder Hosenträgern ausgebildet sein kann, erlaubt eine besonders kleine Bauform der Vorrichtung. Sie hat jedoch den Nachteil, dass die eigentlich vom Schulterbereich abzuleitenden Kräfte an anderer Stelle in den Schulterbereich eingeleitet werden, so dass der Effekt gegenüber der Verwendung eines anderen Gegenlagerelementes reduziert wird. Selbstverständlich kann das Gegenlagerelement auch hier ein flexibles oder starres Element sein, das an der Schulter oder an einem anderen Teil des menschlichen Körpers, insbesondere des Rumpfs des Benutzers, anzuordnen ist.

Es hat sich als vorteilhaft herausgestellt, wenn die Orientierung des Kraftübertragungselementes relativ zu dem Gegenlagerelement durch eine Bewegung des Rumpfes des Benutzers und/oder eine Bewegung des Armes veränderbar ist. Durch diese Veränderung der Orientierung, beispielsweise einer Längserstreckungsrichtung oder einer Winkelstellung des Kraftübertragungselementes relativ zum Gegenlagerelement, lassen sich eine Vielzahl, bevorzugt sogar alle Bewegung und Freiheitsgrade verwirklichen, die nicht durch die schwenkbar Anordnung des Armstützelementes am Druckkraftübertragungselement hervorgerufen werden können. Da die Veränderung der Orientierung einfach durch eine Bewegung des Rumpfes und/oder des Arms des Benutzers hervorgerufen wird, ist eine intuitive Bedienung gewährleistet. Vorteilhafterweise ist das Druckkraftübertragungselement mittels eines Gelenkes, insbesondere eines Kugelgelenkes oder eines Scharniers, mit dem Anlageelement verbunden. Die genaue Ausgestaltung dieses Gelenkes hängt vom Einsatzzweck und den Gegebenheiten, insbesondere auch den individuellen Vorzügen und Vorlieben des Benutzers, ab. Es kann ein Kugelgelenk verwendet werden, das eine Verschwenkung des beispielsweise als Stange oder Stab ausgebildeten Druckkraftübertragungselementes relativ zum Anlageelement, das beispielsweise als Gürtel oder Gurt ausgebildet ist, erlaubt. Auch eine Torsion, also eine Rotation des Druckkraftübertragungselementes um seine eigene Längsachse, kann durch ein derartiges Gelenk erlaubt sein. Es ist jedoch gegebenenfalls ausreichend, ein einfaches Scharniergelenk vorzusehen, mit dem das Druckkraftübertragungselement am Anlageelement angeordnet ist. Dies ist insbesondere dann ausreichend, wenn die Flexibilität des Anlageelementes so groß ist, dass dennoch eine Bewegung des Druckkraftübertragungselementes relativ zum Anlageelement möglich ist, aus der eine Verformung des Anlageelementes folgt. Da das Anlageelement selbst als flexibles Element ausgebildet ist, wird diese Verformung bei einer entsprechenden Bewegung des Armes und/oder Rumpfes des Benutzers rückgängig gemacht. Auf diese Weise ist auch an dieser Stelle eine möglichst einfache Verbindung zwischen dem Druckkraftübertragungselement und dem Anlageelement erreicht.

Das Gelenk, mit dem das Druckkraftübertragungselement am Armstützelement angeordnet ist, kann auf unterschiedliche Weise ausgebildet sein. Es kann als Schub- oder Kettengelenk oder als Klappmechanismus ausgebildet sein. Auch elastische Elemente, beispielsweise Biegefedern, können als Gelenk und gleichzeitig zumindest auch als mechanischer Energiespeicher verwendet werden. Selbstverständlich kann auch das Bowdenzugprinzip, beispielsweise in Form einer Zug-Druck-Gabel, verwendet werden.

Das Armstützelement verfügt über die Armschale, die vorzugsweise an einem Abstandselement angeordnet ist. Dieses Abstandselement ist als Teil des Armstützelementes vorteilhafterweise mit dem Druckkraftübertragungselement oder dem Kraftübertragungselement verbunden. Die Längen des beispielsweise als Schiene oder Stab ausgebildeten Druckkraftübertragungselementes und des gegebenenfalls auch als Stab oder Schiene ausgebildeten Abstandselementes sind dabei vorzugsweise so gewählt, dass der gesamte Winkelbereich der möglichen Bewegung des Oberarms des Trägers abgedeckt wird. Die Armschale ist vorzugsweise gelenkig an dem Abstandselement angeordnet, um einen größtmöglichen Tragekomfort zu erreichen.

In einer bevorzugten Ausgestaltung ist der passive Aktuator eingerichtet, die Kraft in Abhängigkeit von einer Position und/oder einer Orientierung des wenigstens einen Armstützelementes relativ zum Gegenlagerelement, insbesondere in Abhängigkeit von einem Schwenkwinkel des Armstützelementes um die Schwenkachse, aufzubringen, wobei der passive Aktuator die Kraft vorzugsweise exzentrisch auf das Armstützelement aufbringt. Bevorzugt wird die Kraft auf das Abstandselement des Armstützelementes aufgebracht. Anstelle beispielsweise einer Konstantkraftfeder als passiven Aktuator, durch die unabhängig von der Position und Winkelstellung des Oberarms und damit auch des Armstützelementes immer die gleiche Kraft aufgebracht wird, können passive Aktuatoren verwendet werden, durch die je nach Position und/oder Orientierung des Arms und damit des Armstützelementes unterschiedliche Kräfte aufgebracht werden können. So ist es beispielsweise von Vorteil, keine Kraft aufzubringen, sofern der Arm einen bestimmten Winkel relativ zur Normalen, also der der Schwerkraft folgenden Richtung, nicht überschreitet. Dieser Winkel kann beispielsweise 45°, 60° oder 90° sein. Erst wenn der Arm über diesen Winkel hinweg angehoben wird, wird durch den passiven Aktuator vorteilhafterweise eine Kraft aufgebracht, die der Schwerkraft entgegengerichtet ist. Diese Kraft kann mit dem weiteren Anheben, also Verschwenken, des Oberarms und damit des Armstützelementes relativ zum Druckkraftübertragungselement variieren. Dazu können Kurvenscheiben, Getriebe oder andere Kraft-Weg-Funktionen, beispielsweise Kulissen, verwendet werden. Eine maximale Kraft wird bevorzugt in einem Winkelbereich von 70° bis 120°, besonders bevorzugt bei 90° aufgebracht.

Vorzugsweise ist die von dem passiven Aktuator aufbringbare Kraft durch eine veränderbare Vorspannung des passiven Aktuators und/oder eine einstellbare Exzentrizität der Kraftaufbringung auf das Armstützelement veränderbar.

In einer bevorzugten Ausgestaltung ist das Kraftübertragungselement in einer Führung gelagert, durch die verhindert wird, dass sich der Teil des Kraftübertragungselementes, an dem das Armstützelement angeordnet ist, im angelegten Zustand der Vorrichtung vom Rumpf des Benutzers entfernt. Bei der Führung handelt es sich in einer konstruktiv besonders einfachen Ausgestaltung um eine Schlaufe oder eine Hülse, die vorzugsweise aus einem Textil hergestellt sind. Die Führung dient vorzugsweise als Positionierungshilfe. Eine Bewegung des Kraftübertragungselementes relativ zur Führung ist von Vorteil aber nicht notwendig.

Besonders vorteilhafterweise verfügt die Vorrichtung über zwei Armstützelemente zum Abstützen beider Arme, wobei die beiden Armstützelemente jeweils eine Armschale aufweisen und vorzugsweise jeweils an einem Kraftübertragungselement schwenkbar angeordnet sind und zwischen den Kraftübertragungselementen vorteilhafterweise wenigstens ein Verbindungselement, insbesondere ein Zugelement angeordnet ist, durch das vorzugsweise eine Zugkraft auf die beiden Kraftübertragungselemente aufbringbar ist. Auch damit wird verhindert, dass die Kraftübertragungselemente sich mit dem vom Gegenlagerelement abgewandten Ende zu weit vom Körper, insbesondere vom Rumpf, des Benutzers entfernen. Zudem wird vorzugsweise durch ein derartiges Zugelement die Armschale am Arm des Trägers gehalten und ein Verrutschen weitgehend oder vollständig verhindert.

Insbesondere für den Fall zweier Armstützelement und zweier Kraftübertragungselement können diese beispielsweise auch flächig, beispielsweise in Form eines Kreuzes angeordnet werden. Auch mehrere Stützen oder Federn, elastische oder mechanische Energiespeicher oder sonstige Kraftübertragungs- oder Kraftaufbringelemente können verwendet werden. Durch die flächige Anordnung wird einerseits erreicht, dass die Vorrichtung im angelegten Zustand wenig aufträgt, so dass sie bei täglichen Arbeiten wenig stört. Andererseits wird erreicht, dass die Kraftübertragungselemente eher den Bewegungen des Rumpfes des Benutzers folgen.

Vorzugsweise ist die Vorrichtung an unterschiedliche Körpergrößen des Benutzers anpassbar. Auf diese Weise kann die Vorrichtung vielseitig und flexibel eingesetzt werden, ohne dass für unterschiedlich große Benutzer unterschiedliche Vorrichtungen vorgehalten werden müssen. Dabei kann beispielsweise die Länge des Kraftübertragungselementes, insbesondere in Form des Druckkraftübertragungselementes, anpassbar sein, indem beispielsweise Teleskopstangen verwendet werden. Diese können beispielsweise über Schnellspanner oder Dreharretierungen verfügen, so dass sie leicht entriegelt, in der Länge verstellt und wieder verriegelt werden können. Dies geschieht vorzugsweise stufenlos. Aber auch eine stufige Einstellung ist möglich. So können beispielsweise Rastbohrungen vorhanden sein, in die ein von innen federbelasteter Pin einrastet, wenn die gewünschte Länge eingestellt ist. Zum Verstellen der Länge wird der Pin in die Teleskopstange hineingedrückt und die mehreren Elemente der Teleskopstange gegeneinander verschoben.

Selbstverständlich lässt sich auch das Abstandselement des Armstützelementes auf diese Weise in der Länge verstellen. Auch hier kann ein Teleskop oder eine Teleskopstange verwendet werden. Alternativ oder zusätzlich dazu kann die Armschale auf oder in dem Abstandselement gleitend ausgebildet sein, um einen Kongruenzausgleich herbeizuführen. Bei bestimmten Bewegungen des Oberarms relativ zur Schulter kommt es in diesem Fall zu einer Bewegung der Armschale relativ zum Abstandselement, so dass die Armschale immer im gleichen Bereich mit dem Arm des Benutzers in Kontakt kommt.

Alternativ zur Teleskopstange können auch ineinander verdrehte Spiralen oder Drehlager am Oberarm für die Einstellung von Länge und Richtung verwendet werden.

Das Gelenk, mit dem das Kraftübertragungselement am Anlageelement angeordnet ist, kann vorteilhafterweise am jeweiligen Anlageelement fest angeordnet sein. Ist dieses als Gurt oder Gürtel ausgebildet, lässt sich dessen Länge einfach einstellen, wodurch auch die Position des Gelenks einstellbar wird. Das Gelenk, beispielsweise als Clip ausgebildet, kann alternativ oder zusätzlich dazu auch lösbar mit dem Anlageelement verbindbar oder verbunden sein. Alternativ oder zusätzlich dazu kann es entlang einer Schiene, einer Kulisse oder einer sonstigen Verschiebekontur verschiebbar ausgestaltet sein. Weitere Möglichkeiten sind die Anordnung in einer Gürteltasche, die Kopplung des Gelenkes über einen sogenannten Ratschenverschluss, wie er beispielsweise von Skischuhen bekannt ist, als Pinverschluss oder durch einen Klettverschluss.

Besonders einfach lässt sich die passende Größe der Vorrichtung ermitteln, wenn die Vorrichtung in ein Kleidungsstück, insbesondere eine Jacke oder ein Shirt, integriert ist. Insbesondere das Anlageelement ist vorzugsweise in einer Hose integriert. Es ist dann lediglich die passende Konfektionsgröße für den Benutzer zu ermitteln, so dass Kleidungsstücke der passenden Größe ausgewählt werden können.

Da für unterschiedlich große und unterschiedlich kräftige Benutzer unterschiedlich große Kräfte durch den passiven Aktuator aufzubringen sind, hat es sich als vorteilhaft herausgestellt, wenn die Stärke der aufzubringenden Kraft einstellbar ist. Dies kann beispielsweise geschehen, indem eine Vorspannung eines Federelementes, beispielsweise einer Schraubenfeder, beispielsweise über ein Drehrad oder einen Schiebemechanismus einstellbar ist. Auch die entspannte Federlänge, also die Länge der Feder ohne Belastung, kann eingestellt werden. Alternativ oder zusätzlich dazu können mehrere Federstränge oder Federelemente vorhanden sein, die einzeln oder zusammen aktivierbar sind und gegebenenfalls aber nicht notwendigerweise unterschiedliche Federkonstanten und Härten aufweisen. Die Länge eines Hebelarms, an dem die vom passiven Aktuator aufgebrachte Kraft am Armstützelement, insbesondere am Abstandselement des Armstützelementes, angreift, kann ebenfalls eingestellt und beispielsweise über eine Stellschraube verändert werden. Gleiches gilt für den Anschlag eines Bowdenzugs. Die Einstellung kann mechanisch oder mechatronisch, beispielsweise motorgetrieben vornehmbar sein. Die mechatronische Einstellbarkeit ist beispielsweise dann von Vorteil, wenn die aufbringbare Kraft adaptiv anpassbar sein soll, etwa wenn durch Halten eines Gegenstandes, beispielsweise eines Werkzeuges, eine größere Unterstützungskraft notwendig oder gewünscht wird.

Es hat sich als besonders komfortabel herausgestellt, wenn die Federelemente und die anderen mechanischen, hydraulischen oder pneumatischen Energiespeicher erst beim Anziehen der Vorrichtung gespannt werden. Zusätzlich können sie beim Ausziehen entspannt werden. Dies kann beispielsweise durch Hebel, Schlaufen oder lösbare Spannverbindungen geschehen, die mit den jeweiligen Federelementen verbunden sind und ein Spannen der Feder gegebenenfalls bis zum gewünschten Grad erlauben. Alternativ oder zusätzlich dazu können Schieber vorhanden sein, an denen beispielsweise ein Ende eines Federelementes angeordnet ist. Durch Verschieben des Schiebers entlang eines Führungselementes kann die Feder oder das Federelement gespannt oder entspannt werden. Die Arretierung und Feststellung in einer bestimmten Position erfolgt vorteilhafterweise durch Verkanten des Schiebers am Führungselement.

Besonders vorteilhafterweise ist die Armschale eine selbstschließende Schale, die geöffnet ist, solange sich kein Arm in ihr befindet. Wird der Arm in die Schale eingeführt, wird ein Betätigungsmechanismus ausgelöst, durch den sich die Schale schließt. Gleichzeitig können durch diesen Mechanismus die Federelemente gespannt werden.

Vorzugsweise verfügt die Vorrichtung über eine Blockiereinrichtung, durch die eine Bewegung des Armstützelementes relativ zu dem Kraftübertragungselement in wenigstens einer Richtung, vorzugsweise vollständig blockierbar ist.

Vorteilhafterweise befindet sich zwischen den Anstützelementen wenigstens ein Zug-element, insbesondere wenigstens eine Zugfeder. Dadurch wird auf die beiden Armstützelemente jeweils eine Zugkraft in Richtung auf das jeweils andere Armstützelement ausgeübt. Dadurch wird überraschenderweise erreicht, dass die Kraftübertragungselemente sich auch bei komplizierten oder ungewöhnlichen Bewegungen des Trägers der Vorrichtung nicht vom Rumpf des Trägers entfernen. Dadurch wird erreicht, dass die Gefahr, mit Bauteilen der Vorrichtung beispielsweise an Gegenständen oder Türrahmen anzustoßen, was die Akzeptanz der Vorrichtung verringern würde, reduziert wird.

Vorzugsweise verfügt wenigstens eine der Armschalen, bevorzugt jedoch beide Armschalen, über ein Verschlusselement, das insbesondere als Gurt ausgebildet sein kann. Durch dieses Verschlusselement ist die Armschale um den Arm schließbar. Der Gurt kann beispielsweise um den Arm herumgelegt und mit einem Befestigungselement, das an einem freien Ende des Gurtes angeordnet und beispielsweise in Form eines Klettverschlusses ausgebildet sein kann, an dem gegenüberliegenden Ende der Armschale befestigt werden. Selbstverständlich sind auch andere Formen der Befestigung, beispielsweise eine Schnalle, ein Formschlusselement, beispielsweise in Form eines Druckknopfes, oder andere Verschlusselemente möglich.

Vorteilhafterweise ist das Verschlusselement mittels wenigstens eines Befestigungselementes an einem anderen Element als der Armschale der Vorrichtung befestigbar. Dabei ist dieses andere Element vorteilhafterweise ein Schultergurt oder ein Kraftübertragungselement oder eine Umhüllung eines dieser Kraftübertragungselemente oder das Gegenlagerelement. Soll in diesem Fall der Träger der Vorrichtung die Vorrichtung ablegen, muss er zunächst das Verschlusselement lösen, um seinen Arm aus der Armschale entnehmen zu können. Herkömmlicherweise besteht in diesem Moment die Gefahr, dass durch das Entfernen des Armes aus der Armschale die durch den Arm auf das Armstützelement aufgebrachte Kraft reduziert wird, während die vom passiven Aktuator aufgebrachte Kraft weiterhin vorhanden ist. Es kann in diesem Fall zu einer schnellen, beispielsweise ruckartigen, Bewegung des Armstützelementes nach oben kommen, was zu Verletzungen oder Beschädigungen umstehender Geräte oder Gegenstände führen kann. Ist jedoch die Armschale mit dem Verschlusselement verschlossen, muss das Verschlusselement zunächst gelöst werden, um den Arm aus der Armschale entnehmen zu können. Der Träger der Vorrichtung hält folglich ein Ende des Verschlusselementes in der Hand und kann auf diese Weise weiterhin eine nach unten wirkende Kraft auf die Armschale und damit auf das Armstützelement aufbringen. Das Verschlusselement wird in diesem Fall vorzugsweise an dem anderen Element der Vorrichtung, beispielsweise einem Schultergurt, angeordnet. Die Vorrichtung wird auf diese Weise kontrolliert und gegebenenfalls langsam in eine Art "Parkposition" gebracht, ohne dass es zu einer rückartigen Bewegung kommt.

Beim Anlegen einer derartigen Vorrichtung wird umgekehrt verfahren. Der Träger der Vorrichtung, der die Vorrichtung anlegen will, löst zunächst das Verschlusselement von dem anderen Element der Vorrichtung und kann auf diese Weise bereits eine Kraft auf das Armstützelement aufbringen, um dieses in die zum Anlegen gewünschte Position zu bringen. Dabei muss er das Verschlusselement vorteilhafterweise nicht loslassen, bis sich der Arm in der Armschale befindet. Anschließend wird das Verschlusselement um den Arm geschlossen und die Vorrichtung ist sicher angelegt.

Vorteilhafterweise verfügt die Vorrichtung über einen Anschlag, durch den eine Bewegung des Armstützelementes relativ zu dem Druckkraftübertragungselementes um die Schwenkachse herum begrenzt wird. Vorteilhafterweise verfügt die Vorrichtung über zwei Anschläge, um die Bewegung jeweils eines Armstützelementes um die Schwenkachse relativ zu dem jeweiligen Druckkraftübertragungselementes zu begrenzen. Auf diese Weise wird verhindert, dass beispielsweise nach Überschreiten der Lotrechten es zu einem Überschlagen der verschiedenen Bauteile relativ zueinander kommt.

Vorzugsweise ist der Anschlag in eine aktive Position, in der er die Bewegung begrenzt, und in eine passive Position bringbar, in der er die Position nicht begrenzt.

Dies geschieht vorteilhafterweise dadurch, dass der Anschlag ein Vorsprung an dem Druckkraftübertragungselement ist. In diesem Fall kann das Druckkraftübertragungselement einfach um seine Längsachse rotiert werden, um den Vorsprung in die aktive oder die passive Position zu bringen.

Vorzugsweise verfügt das Gegenlagerelement über einen durch einen Verschluss verschließbaren Gurt, der um einen Rumpf des Trägers herumlegbar ist. Ein solcher Gurt stellt eine eigenständige Erfindung dar und ist allein oder als Teil einer Vorrichtung, insbesondere als Teil einer Orthese oder einer Prothese einsetzbar. So kann ein solcher Gurt auch um ein anderes Körperteil eines Trägers einer Vorrichtung oder einen Gegenstand herumlegbar sein. Ein solcher Gurt ist beispielsweise immer dann von Vorteil, wenn eine Spannung auf den Gurt im geschlossenen Zustand wirkt, so dass ein versehentliches Öffnen des Gurtes zu einem ruckartigen und unkontrollierten Lösen dieser Spannung führen würde. Dabei handelt es sich beispielsweise um einen Hüft- oder einen Bauchgurt. Der Verschluss ist dabei so ausgebildet, dass wenigstens zwei Schritte zum Öffnen und Schließen des Verschlusses notwendig sind. Wird als Verschluss beispielsweise lediglich ein Schnappverschluss verwendet, der beispielsweise durch Betätigen eines oder zweier Betätigungselemente gelöst werden kann, besteht die Gefahr, dass aufgrund der durch die Aktuatoren wirkenden Kräfte die Enden des Gurtes voneinander weg beschleunigt werden, um gegen umgebende Gegenstände oder Personen geschlagen werden. Um dies zu verhindern ist der Verschluss zweistufig ausgebildet.

Vorteilhafterweise muss ein Ende des Gurtes durch eine am anderen Ende angeordnete Öse geführt werden, bevor ein Formschluss zweier korrespondierend ausgebildeter Formschlusselemente hergestellt wird, um den Verschluss zu schließen. Dabei befindet sich an dem Ende, das durch die Öse geführt wird, vorteilhafterweise eines der beiden Formschlusselemente. Zum Öffnen des Verschlusses muss zunächst der Formschluss gelöst und dann eines der Enden des Gurtes durch die besagte Öse geführt werden, wobei wieder das eine Formschlusselement durch die Öse geführt wird.

Vorteilhafterweise verfügt der passive Aktuator über wenigstens ein elastisches Element und die Vorrichtung über wenigstens ein Anlageelement, das bei einem vorbestimmten Winkel zwischen dem Armstützelement und dem Kraftübertragungselement an dem elastischen Element anliegt und so die auf das Armstützelement aufzubringende Kraft verändert. Das elastische Element übt beim Bewegen des Armstützelementes relativ zum Kraftübertragungselement eine Kraft aus, da es gedehnt oder gestaucht wird. Vorzugsweise ist das Abstützelement relativ zum Kraftübertragungselement drehbar gelagert und ein Angriffspunkt der durch das elastische Element aufgebrachten Kraft ist bezüglich der Drehachse des entsprechenden Gelenkes exzentrisch gelagert. Beim Verschwenken des Armstützelementes relativ zum Kraftübertragungselement verändert sich daher die Richtung der durch das elastische Element aufgebrachten Kraft. Das Anlageelement, das vorteilhafterweise am Gelenk angeordnet ist, ist dabei insbesondere so angeordnet, dass es bei einem vorbestimmten Winkel mit dem elastischen Element in Kontakt kommt. Ab diesem Moment wird die auf den Angriffspunkt wirkende Kraft nicht mehr aus der Richtung zwischen dem Angriffspunkt und dem zweiten Lagerpunkt des elastischen Elementes sondern durch die Richtung zwischen dem Angriffspunkt und dem Punkt bestimmt, an dem das Anlageelement am elastischen Element anliegt. Ist dieser Punkt möglichst nahe an der Rotationsachse, vorzugsweise genau an der Rotationsachse, wird die Kraft, die durch den Teil des elastischen Elementes aufgebracht wird, nahezu oder genau axial verlaufen, so dass kein Drehmoment mehr auf das Armstützteil ausgeübt wird. Durch eine Verschiebung oder Veränderung der Position des Anlageelementes kann diese Kraft eingestellt werden.

Vorteilhafterweise ist daher der vorbestimmte Winkel und/oder die Veränderung der aufzubringenden Kraft einstellbar, indem vorteilhafterweise das Anlageelement relativ zu dem Armstützelement und/oder dem Kraftübertragungselement verschiebbar ist. Dies kann beispielsweise geschehen, indem das Anlageelement in einer Kulisse verschiebbar und beispielsweise durch eine Schraube fixierbar ist.

Vorliegend werden insbesondere Vorrichtungen offenbart, deren Ausführungsformen durch folgende Merkmalskombinationen beschrieben werden.
1. Vorrichtung zum Unterstützen zweier Arme (4) eines Benutzers (2), wobei die Vorrichtung
   - zwei Armstützelemente (6) mit je einer Armschale (10) zum Anlegen an jeweils einen Arm (4),
   - wenigstens einen passiven Aktuator (26),
      o der eingerichtet ist, eine Kraft auf wenigstens eines der Armstützelemente (6) aufzubringen, und
   - wenigstens ein Gegenlager (14) für die aufzubringende Kraft aufweist, das
      o wenigstens ein Gegenlagerelement (16) und
      o wenigstens zwei Kraftübertragungselemente (18) aufweist, die eingerichtet sind, eine Gegenkraft von jeweils einem der Armstützelemente auf das Gegenlagerelement (16) zu übertragen,
      **dadurch gekennzeichnet, dass** die Kraftübertragungselemente (18) relativ zu dem Gegenlagerelement (16) bewegbar, insbesondere um wenigstens eine Rotationsachse drehbar, an dem Gegenlagerelement (16) angeordnet sind.
2. Vorrichtung nach Ausführungsform 1, **dadurch gekennzeichnet, dass** das Kraftübertragungselement (18) ein Druckkraftübertragungselement, insbesondere eine Stange oder eine Schiene ist, an dem das Armstützelement (6) um eine Schwenkachse schwenkbar angeordnet ist.
3. Vorrichtung nach Ausführungsform 1 oder 2, **dadurch gekennzeichnet, dass** sich zwischen den Armstützelementen (6) wenigstens ein Zugelement (58), insbesondere wenigstens eine Zugfeder, befindet.
4. Vorrichtung nach einer der vorstehenden Ausführungsformen, **dadurch gekennzeichnet, dass** wenigstens eine der Armschalen (10) ein Verschlusselement (54), insbesondere einen Gurt, aufweist, durch den die Armschale (10) um den Arm schließbar ist.
5. Vorrichtung nach Ausführungsform 4, **dadurch gekennzeichnet, dass** das Verschlusselement (54) mittels wenigstens eines Befestigungselementes (60) an einem anderen Element der Vorrichtung als der Armschale (10), insbesondere an einem Schultergurt (30) oder einem der Kraftübertragungselemente (18) oder eine Umhüllung (56) wenigstens eines der Kraftübertragungselemente (18) oder an dem Gegenlagerelement (16) befestigbar ist.
6. Vorrichtung nach einer der Ausführungsformen 2 bis 5, **dadurch gekennzeichnet, dass** die Vorrichtung einen Anschlag (80) aufweist, durch den eine Bewegung eines Armstützelementes (6) relativ zu dem Druckkraftübertragungselement um die Schwenkachse begrenzt wird.
7. Vorrichtung nach Ausführungsform 6, **dadurch gekennzeichnet, dass** der Anschlag (80) in eine aktive Position, in der er die Bewegung begrenzt, und in eine passive Position bringbar ist, in der er die Bewegung nicht begrenzt.
8. Vorrichtung nach Ausführungsform 7, **dadurch gekennzeichnet, dass** der Anschlag (80) ein Vorsprung (80) an dem Druckkraftübertragungselement ist.
9. Vorrichtung nach einer der vorstehenden Ausführungsformen, **dadurch gekennzeichnet, dass** der passive Aktuator (26) die Kraft in Abhängigkeit von einer Position und/oder Orientierung des wenigstens einen Armstützelementes (6) relativ zu dem Gegenlagerelement (16), insbesondere in Abhängigkeit von einem Schwenkwinkel des Armstützelementes (6) um die Schwenkachse, aufbringt, wobei der passive Aktuator (26) die Kraft vorzugsweise exzentrisch auf das Armstützelement (6) aufbringt.
10. Vorrichtung nach einer der vorstehenden Ausführungsformen, **dadurch gekennzeichnet, dass** die von dem passiven Aktuator (26) aufbringbare Kraft durch eine veränderbare Vorspannung des passiven Aktuators (26) und/oder eine einstellbare Exzentrizität der Kraftaufbringung auf das Armstützelement (6) veränderbar ist.
11. Vorrichtung nach einer der vorstehenden Ausführungsformen, **dadurch gekennzeichnet, dass** das Gegenlagerelement (16) einen durch einen Verschluss (66) verschließbaren Gurt aufweist, der um einen Rumpf des Trägers herumlegbar ist, wobei der Verschluss (60) derart ausgebildet ist, dass wenigstens zwei Schritte zum Öffnen und Schließen des Verschlusses (60) notwendig sind.
12. Vorrichtung nach Ausführungsform 11, **dadurch gekennzeichnet, dass** ein Ende des Gurtes durch eine Öse (76) geführt und anschließend ein Formschluss zweier korrespondierend ausgebildeter Formschlusselemente hergestellt werden muss, um den Verschluss (66) zu schließen und der Formschluss gelöst und das Ende des Gurtes durch die Öse (76) geführt werden muss, um den Verschluss (66) zu öffnen.
13. Vorrichtung nach einer der vorstehenden Ausführungsformen, **dadurch gekennzeichnet, dass** der passive Aktuator (26) wenigstens ein elastisches Element aufweist und die Vorrichtung wenigstens ein Anlageelement (78) aufweist, das bei einem vorbestimmten Winkel zwischen dem Armstützelement (6) und dem Kraftübertragungselement (18) an dem elastischen Element anliegt und so die auf das Armstützelement (6) aufzubringende Kraft verändert.
14. Vorrichtung nach Ausführungsform 13, **dadurch gekennzeichnet, dass** der vorbestimmte Winkel und/oder die Veränderung der aufzubringenden Kraft einstellbar ist, indem vorzugsweise das Anlageelement (78) relativ zu dem Armstützelement (6) und/oder zu dem Kraftübertragungselement (18) verschiebbar ist.

Mit Hilfe der beiliegenden Zeichnungen wird nachfolgend ein Ausführungsbeispiel der vorliegenden Erfindung näher erläutert. Es zeigen:
- Figur 1 -: eine schematische Darstellung einer Vorrichtung gemäß einem ersten Ausführungsbeispiel der vorliegenden Erfindung im angelegten Zustand,
- Figuren 2 - 6 -: eine Darstellung einer angelegten Vorrichtung in unterschiedlichen Positionen in einer Seitenansicht,
- Figuren 7 - 9 -: Darstellungen einer angelegten Vorrichtung in einer Draufsicht von oben,
- Figur 10 -: eine schematische Darstellung einer Vorrichtung gemäß einem weiteren Ausführungsbeispiel der vorliegenden Erfindung,
- Figur 11 -: eine schematische Darstellung eines Ausschnittes einer Vorrichtung,
- Figuren 12 - 15 -: schematische Darstellungen von Vorrichtungen gemäß weiterer Ausführungsbeispiele der vorliegenden Erfindung,
- Figuren 16 und 17 -: beispielhafte Darstellungen von Kraftverläufen,
- Figur 18 -: die schematische Darstellung einer angelegten Vorrichtung,
- Figuren 19 und 20 -: schematische Darstellungen einzelner Elemente der Vorrichtung,
- Figur 21 -: verschiedene Schritte zum Lösen eines Gurtes,
- Figuren 22 und 23 -: verschiedene Formen von Anschlägen,
- Figuren 24 bis 26 -: unterschiedliche Arten, eine aufgebrachte Kraft einzustellen und
- Figuren 27 und 28 -: weitere Ausführungsformen der Erfindung.

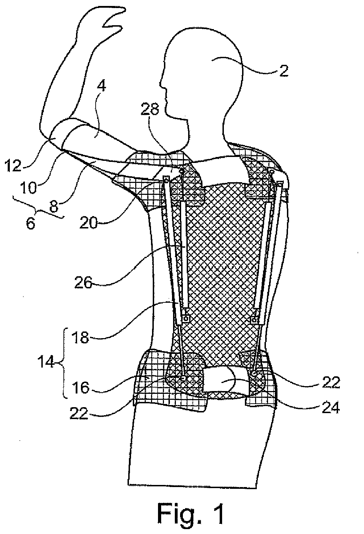

Figur 1 zeigt einen Benutzer 2, der eine Vorrichtung zum Unterstützen eines Armes 4 trägt. Die Vorrichtung verfügt über ein Armstützelement 6, das ein Abstandselement 8 und eine Armschale 10 aufweist. An deren distalem Ende ist das Armstützelement 6 über eine Manschette 12 am Arm 4 angeordnet.

Die Vorrichtung verfügt zudem über ein Gegenlager 14, das ein Gegenlagerelement 16 und ein Kraftübertragungselement 18 aufweist. Das Abstandselement 8 des Armstützelementes 6 ist über ein Gelenk 20 an dem in Figur 1 oberen Ende des Kraftübertragungselementes 18 angeordnet. Dieses ist beispielsweise in Form einer Stange ausgebildet. Im gezeigten Ausführungsbeispiel ist das Kraftübertragungselement 18 eine Teleskopstange. Das Kraftübertragungselement 18 ist mit seinem unteren Ende im gezeigten Ausführungsbeispiel über ein Kugelgelenk 22 am Gegenlagerelement 16 angeordnet. Das Gegenlagerelement 16 dient dazu, die auftretenden Kräfte in ein stabiles Element zu übertragen. Es kann an einem Boden, einem Gegenstand oder einem Körperteil anliegen. Im gezeigten Ausführungsbeispiel ist das Gegenlagerelement 16 ein Hüftgurt, der im Hüftbereich am Benutzer 2 anliegt. Über eine Einstellvorrichtung 24 lässt sich die Länge des Gegenlagerelementes 16 einstellen. Dies ist einerseits für den Komfort für den Benutzer 2 und andererseits für eine möglichst exakte Positionierung der Kugelgelenke 22 am Körper des Benutzers 2 von Vorteil.

Die in Figur 1 gezeigte Vorrichtung verfügt zudem über einen passiven Aktuator 26, der im gezeigten Ausführungsbeispiel in Form einer Zugfeder ausgebildet ist. Das in Figur 1 untere Ende des passiven Aktuators 26 ist am Kraftübertragungselement 18 angeordnet, während das gegenüberliegende Ende an einem Hebelelement 28 des Abstandselementes 8 des Armstützelementes 6 angreift. Durch den passiven Aktuator 26 wird eine Kraft auf das Hebelelement 28 und damit auf das Abstandselement 8 des Armstützelementes 6 aufgebracht, die der Schwerkraft entgegenwirkt und den Arm 4 des Benutzers 2 stützt.

Die in Figur 1 gezeigte Vorrichtung verfügt über zwei Armstützelemente 6, zwei Gegenlager 14 und zwei passive Aktuatoren 26, von denen der Übersichtlichkeit halber nur einer beschrieben wurde.

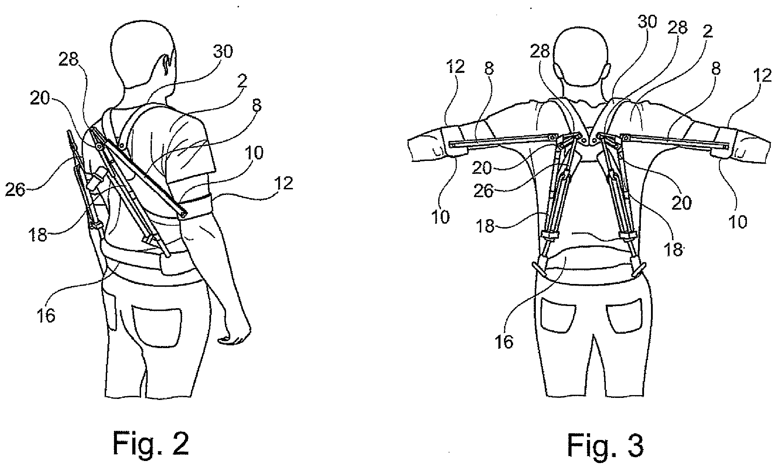

Die Figuren 2 bis 6 zeigen eine Vorrichtung gemäß einem Ausführungsbeispiel der vorliegenden Erfindung im angelegten Zustand, wobei der Benutzer 2 unterschiedliche Bewegungen ausgeführt hat. Figur 2 zeigt das entspannte Stehen des Benutzers 2, während in Figur 3 der Benutzer 2 mit ausgebreiteten Armen zu erkennen ist. Die jeweilige Armschale 10 ist über eine Manschette 12 am Arm festgelegt und bildet gemeinsam mit dem Abstandselement 8 das Armstützelement 6. Es ist über das Gelenk 20 mit dem Kraftübertragungselement 18 verbunden, wobei die jeweiligen Kraftübertragungselemente 18 am Gegenlagerelement 16 in Form des Hüftgurtes angeordnet sind. Am Gelenk 20 befindet sich das Hebelelement 28, an dem der passive Aktuator 26 angreift.

In Figur 2 beim entspannten Stehen ist deutlich zu erkennen, dass das Gelenk 20 sich nach hinten vom Körper des Benutzers 2 entfernt. Breitet der Benutzer 2 wie in Figur 3 dargestellt die Arme aus, verschiebt sich das jeweilige Gelenk 20 an den Körper des Benutzers 2 heran. Das Gelenk 20 befindet sich im gezeigten Ausführungsbeispiel im Bereich des Schultergelenks. Die Vorrichtung verfügt zudem über zwei Schultergurte 30, durch die die Vorrichtung am Körper gehalten wird. Sie dienen nicht dazu, die Gelenke 20 in einem vorbestimmten Abstand zum Körper zu halten.

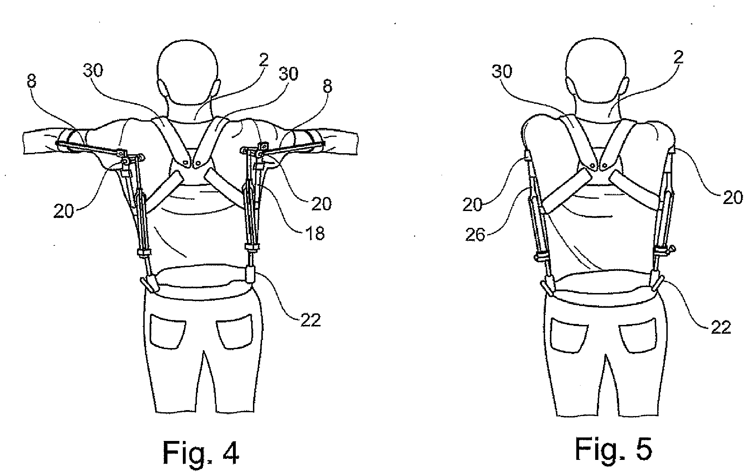

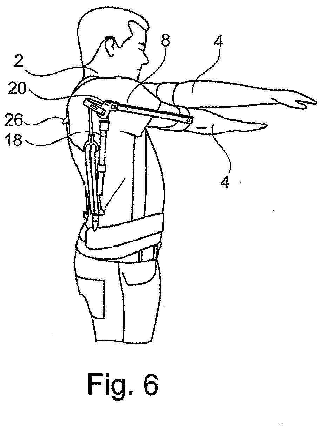

Figur 4 zeigt im Vergleich zu Figur 3 ein weiteres Anheben der Arme. Zudem werden die Arme in den Figuren 4 und 5 vom Benutzer 2 weiter nach vorne geführt. Man erkennt, dass sich die beiden Gelenke 20 voneinander entfernen und insbesondere nicht mit den Schultergurten 30 verbunden sind, um in einem vorbestimmten Abstand gehalten zu werden. Die Gelenke 20 sowie die Kraftübertragungselemente 18 und die Abstandselemente 8, die durch die Gelenke 20 miteinander verbunden werden, bewegen sich vielmehr nach außen und sind nahezu völlig frei bewegbar. Man erkennt, dass die Vorrichtung ohne komplizierte Fehlgelenksmechaniken oder starre Schienensysteme auskommt. Während sich die Gelenke 20 in den Figuren 3 und 4 noch hinter dem Körper des Benutzers 2 im Bereich des Schulterblattes befinden, sind sie in Figur 5 allein durch die Bewegung des Körpers an die Seiten des Benutzers 2 gewandert und können so der Bewegung folgen. Figur 6 zeigt die in Figur 5 dargestellte Position in einer Seitenansicht. Die Arme 4 des Benutzers 2 überkreuzen sich, und die Gelenke 20 befinden sich in der gezeigten Darstellung in Figur 6 neben der Schulter des Benutzers 2. Gleichzeitig sind die Kugelgelenke 22, mit denen das Kraftübertragungselement 18 am Gegenlagerelement 16 angeordnet ist, nicht verändert worden. Die Position dieser Kugellager 22 bleibt unabhängig von der Bewegung der Arme 4 des Benutzers 2 unverändert. Eine derartige Bewegungsfreiheit bei derartig geringen konstruktivem Aufwand ist mit Vorrichtungen aus dem Stand der Technik kaum möglich.

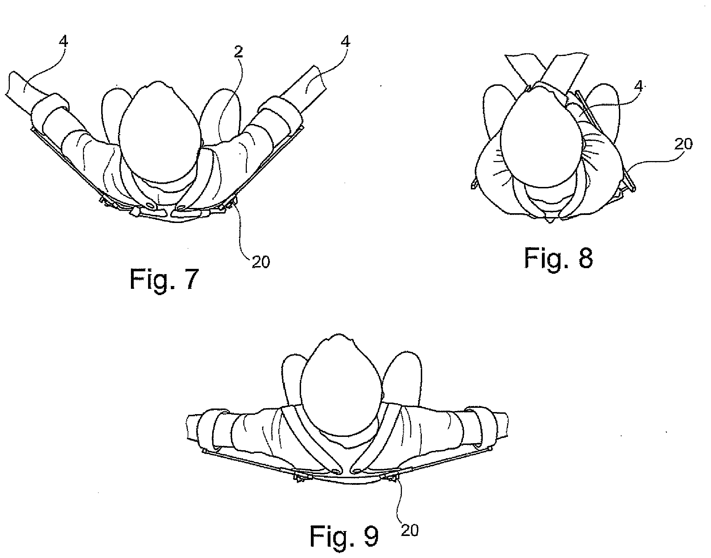

Die Figuren 7 bis 9 zeigen verschiedene Positionen des Benutzers 2 und seiner Arme 4 in einer Draufsicht. In Figur 9 sind die Arme weit ausgebreitet und werden über die Positionen in den Figuren 7 und 8 nach vorne geführt, bis sie sich in Figur 8 überkreuzen. Man erkennt, wie die Gelenke 20 von Figur 9 über Figur 7 nach Figur 8 nach außen wandern und sich dabei immer näher an den Körper des Benutzers 2 anschmiegen.

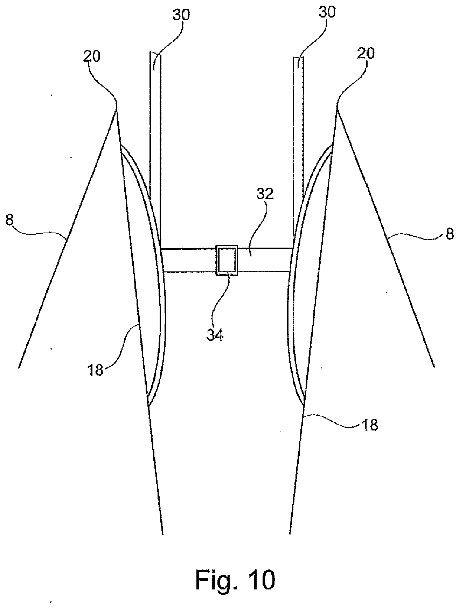

Figur 10 zeigt die schematische Darstellung einer Vorrichtung gemäß einem Ausführungsbeispiel. Man erkennt die Abstandselemente 8, die über Gelenke 20 mit den Kraftübertragungselementen 18 verbunden sind. Anders als in den in den Figuren 2 bis 9 gezeigten Ausführungsbeispielen befindet sich zwischen den beiden Kraftübertragungselementen nun ein Verbindungselement 32, das im gezeigten Ausführungsbeispiel als elastischer Gurt ausgebildet ist. Über eine Schnalle 34 lässt sich die Länge des Gurtes einstellen, sodass die aufbringbare Zugkraft einstellbar ist.

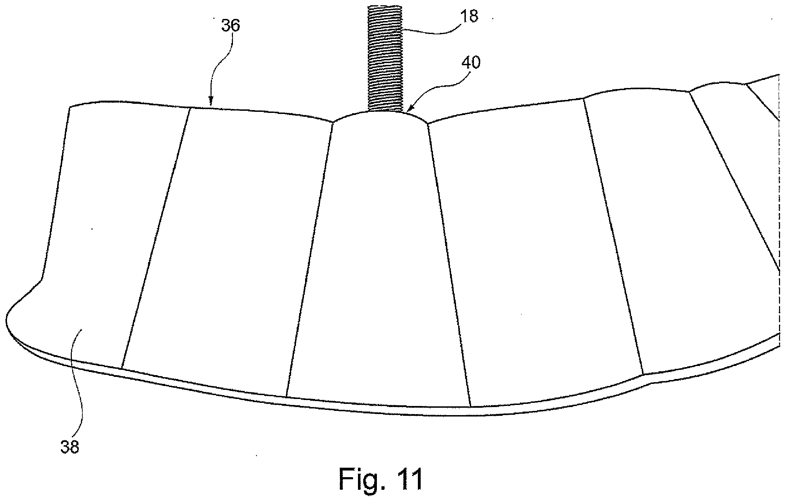

Die beiden Schultergurte 30 sind ebenfalls dargestellt, mit denen die Vorrichtung 1 am Körper des Benutzers 2 angeordnet werden können. Nicht dargestellt ist das Gegenlagerelement. Dieses ist in Figur 11 gezeigt. Es umfasst einen weiteren Gurt 36, der an dem Körper zugewandten Seite über eine Polsterung 38 verfügt, um einen möglichst hohen Tragekomfort zu erreichen. Das Kraftübertragungselement 18 ist in einer dafür vorgesehene Tasche 40 eingesetzt und kann in dieser verschwenkt werden, sodass die Funktion eines Kugelgelenks erfüllt wird. Auf diese konstruktiv besonders einfache Weise kann auf komplizierte Gelenkanordnungen verzichtet werden.

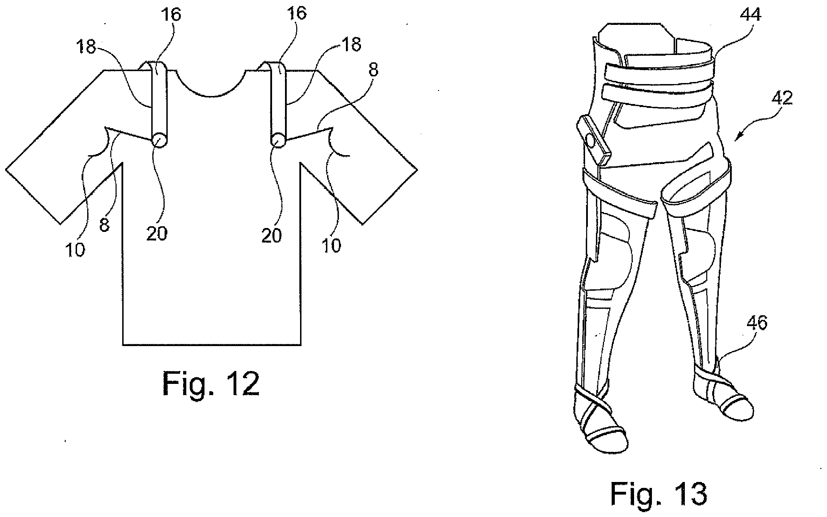

Figur 12 zeigt die schematische Darstellung, bei der die Vorrichtung in ein Bekleidungsstück, vorliegend ein T-Shirt, integriert wurde. Das Gegenlagerelement 16 besteht nun aus zwei Schulterelementen, die über Kraftübertragungselemente 18 und entsprechende Gelenke 20 mit den Abstandselementen 8 und den Armschalen 10 verbunden sind. Dadurch, dass die Vorrichtung in ein T-Shirt integriert ist, lässt sie sich besonders leicht anlegen und ausziehen, sodass keine komplizierten Handgriffe nötig sind. Dadurch wird die Akzeptanz der Vorrichtung gesteigert.

Figur 13 zeigt ein Exoskelet 42, das verschiedene Schienen und Gelenke aufweist, durch die eine Kraft von einem Hüftelement 44 in dafür vorgesehene Bodenkontakt-elemente 46 eingeleitet werden kann.

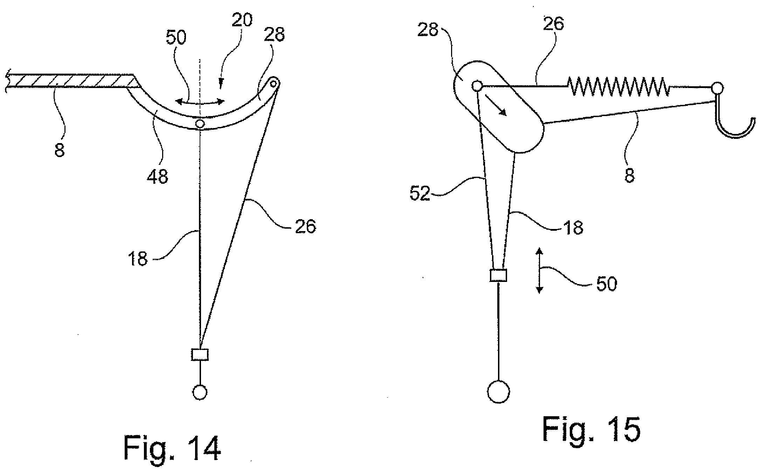

Figur 14 zeigt die schematische Darstellung einer anderen Ausgestaltung des Gelenks 20. Ein Schwenkhebel 48 verbindet das Abstandselement 8 und das Kraftübertragungselement 18, dessen in Figur 14 oberes Ende in einem nicht gezeigten Langloch oder einer Kulisse verschieblich angeordnet ist. Beim Verschwenken verschiebt sich nun der Anlenkungspunkt des Kraftübertragungselementes 18 am Schwenkhebel 48 in Richtung des Doppelpfeiles 50. Dadurch verändert sich auch die Länge des Hebelelementes 28, sodass die durch den passiven Aktuator 26 aufgebrachte Kraft sich ebenfalls verändert.

Figur 15 zeigt eine weitere Ausgestaltung, bei der nun anders als bei den bisherigen Ausführungsformen der passive Aktuator 26 nicht zwischen dem Hebelelement 28 und dem Kraftübertragungselement 18, sondern zwischen dem Hebelelement 28 und dem Abstandselement 8 angeordnet ist. Über einen Gegenspanner 52, dessen Position am Kraftübertragungselement 18 entlang des Doppelpfeiles 50 verschiebbar ist, wird eine Vorspannung auf den passiven Aktuator 26 aufgebracht.

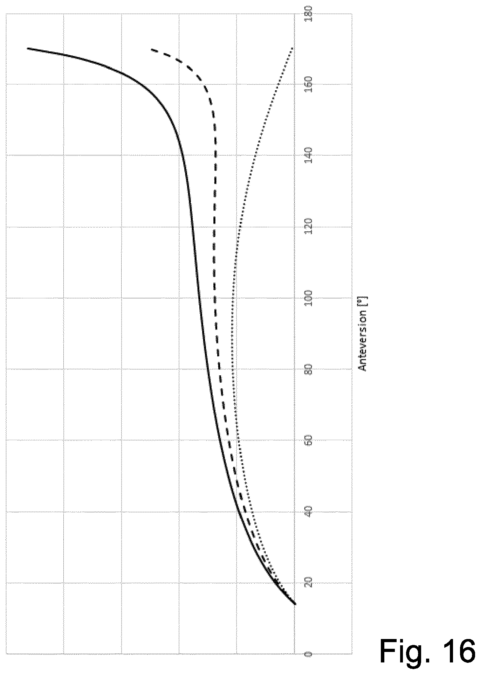

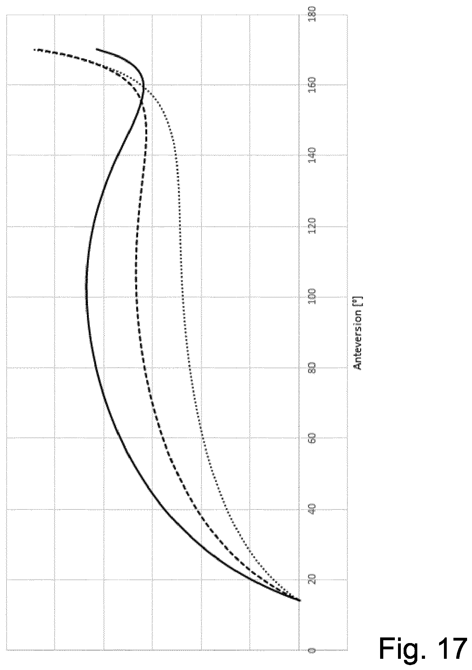

Die Figuren 16 und 17 zeigen unterschiedliche Kraftverläufe, die durch die Vorrichtung gemäß Ausführungsbeispielen der vorliegenden Erfindung auf den Arm aufgebracht werden können. Auf der X-Achse ist ein Anteversionswinkel aufgetragen. Die Anteversion ist das Anheben eines Armes in einer Sagittalebene, also im vorliegenden Beispiel nach vorn. 0° entsprechen dem schlaff nach unten hängenden Arm. Man erkennt in Figur 16, dass erst ab einem vorbestimmten Winkel überhaupt eine Kraft aufgebracht wird. Auf der Y-Achse ist ein Drehmomenten-Verhältnis aufgetragen. Das durch das Eigengewicht des Arms und die Gewichtskraft hervorgerufene Drehmoment wurde durch das durch die Vorrichtung ausgebrachte Drehmoment geteilt.

Die verschiedenen Kraftverläufe sind nahezu individuell einstellbar und im gezeigten Ausführungsbeispiel durch einen einzigen passiven Aktuator 26 erzeugt worden, der in unterschiedlichem Maße vorgespannt ist. Während die gepunktete Linie mit einem passiven Aktuator 26 erzeugt wurde, der nahezu keine Vorspannung aufweist, nimmt die Vorspannung über die gestrichelte Linie und die durchgezogene Linie zu.

Figur 17 zeigt ebenfalls die auf den Arm aufbringbare Kraft über dem Winkel einer Anteversion, wobei nun jedoch die Vorspannung des Aktuators unverändert bleibt. Stattdessen wird ein Anlenkungspunkt verschoben, über den der passive Aktuator 26 seine Kraft auf das am Stützelement 6 überträgt. Dabei nimmt der Abstand des Kraftanlenkungspunktes vom Drehpunkt des Gelenkes 20 von der gepunkteten Linie über die gestrichelte Linie zur durchgezogenen Linie hinzu. Man erkennt, dass auch auf diese Weise eine Erhöhung der Kraft über einen weiten Winkelbereich erreicht werden kann, insbesondere bei großen Winkeln ab ca. 150° jedoch ein deutlich anderer Verlauf auftritt, als dies bei den in Figur 16 dargestellten Verläufen der Fall ist.

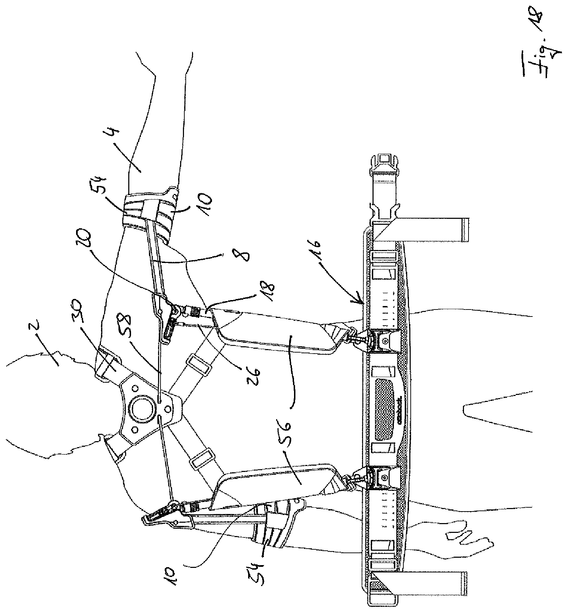

Figur 18 zeigt die Vorrichtung im beinah angelegten Zustand. Das Gegenlagerelement 16 in Form eines Hüftgurtes ist noch geöffnet, während die Armschalen 10 bereits mit einem Verschlusselement 54 um den Arm 4 des Benutzers 2 geschlossen sind. Die Abstandselemente 8 sind über ein Gelenk 20 an dem jeweiligen Kraftübertragungselement 18 angeordnet, das in einer Umhüllung 56 angeordnet ist. Der passive Aktuator 26 ist in Form eines elastischen Seiles oder Drahtes ausgebildet. An den Abstandselementen 8 ist ein Zugelement 58 angeordnet, das die beiden Abstandselemente 8 und damit die beiden Armstützelemente 6 miteinander verbindet. Die Vorrichtung verfügt zudem über Schultergurte 30, durch deren Verbindungselement das Zugelement 58 geführt ist.

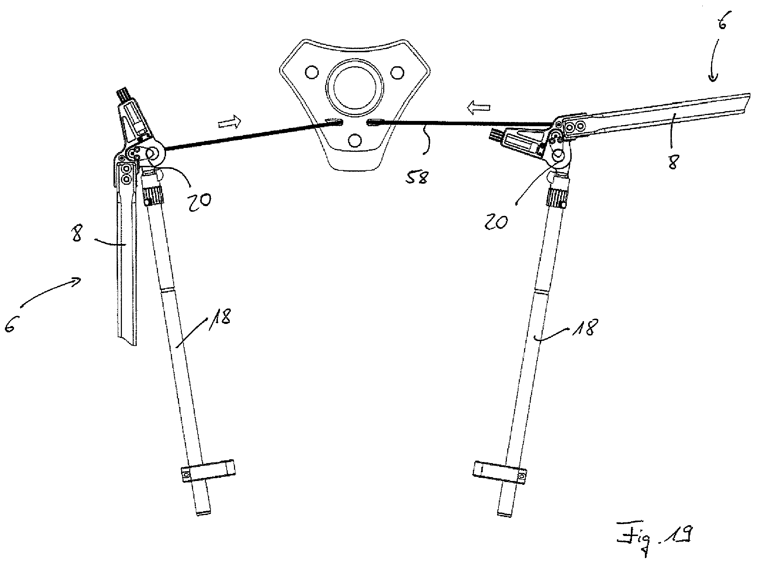

Elemente dieser Ausgestaltung sind in Figur 19 dargestellt. An den beiden Kraftübertragungselementen 18 ist jeweils ein Abstandselement 8 jeweils eines Armstützelement 6 um jeweils ein Gelenk 20 herum schwenkbar angeordnet. Zwischen den beiden Abstandselementen 8 befindet sich das Zugelement 58, das durch das Verbindungselement zwischen den Schultergurten, die nicht dargestellt sind, hindurchverläuft.

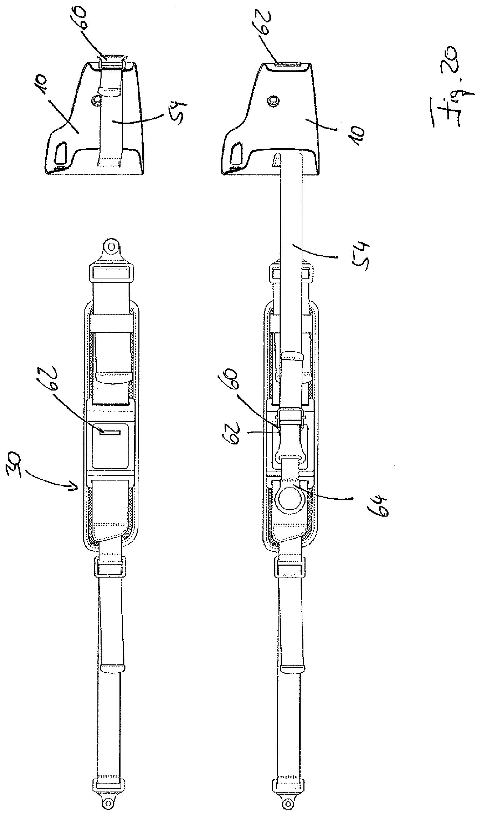

Figur 20 zeigt im oberen Bereich einen der Schultergurte 30 sowie eine Armschale 10 mit einem daran angeordneten Verschlusselement 54. Das Verschlusselement 54 verfügt über ein Befestigungselement 60 in Form einer Schnalle, die mit einer im unteren Teil der Figur 20 gezeigten Öse 62 zusammenwirken kann, um die Armschale 10 um einen nicht dargestellten Arm herum zu schließen. Der Schultergurt 30 verfügt über eine analog ausgebildete Öse 62. Im unteren Bereich der Figur 20 ist das Verschlusselement 54 in geöffneten Zustand dargestellt. Die Armschale 10 ist nicht mehr vollständig um den Arm herum geschlossen. Das Befestigungselement 60 ist nun in die Öse 62 des Schultergurtes 30 eingesetzt, und kann auf diese Weise sicher gelagert werden. Am Ende des Verschlusselementes 54 befindet sich ein Griffelement 64, durch das das Verschlusselement 54 leicht greifbar ist.

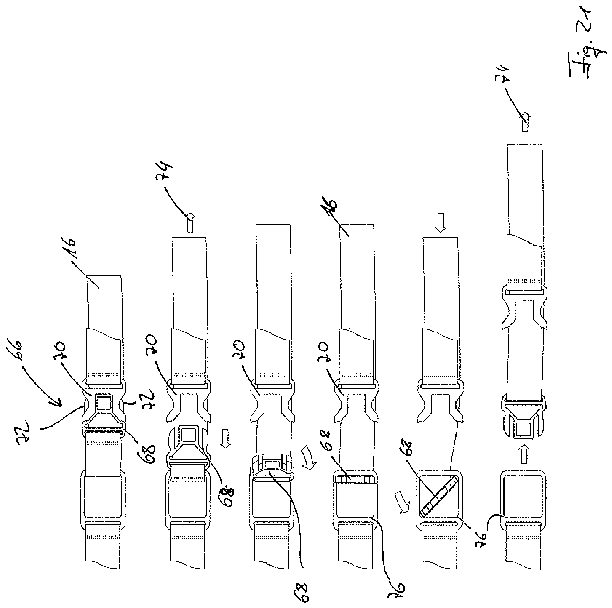

Figur 21 zeigt die verschiedenen Phasen beim Öffnen eines Verschlusses 66 des Gegenlagerelementes 16, das beispielsweise als Hüftgurt ausgebildet ist. Ein solcher Gurt ist auch für andere Gegenstände von Vorteil und stellt allein oder als Teil einer Vorrichtung, insbesondere einer Orthese oder Prothese eine eigenständige Erfindung dar. Die oberste Darstellung zeigt den Verschluss 66 im geschlossenen Zustand. Er verfügt über eine Schnalle, deren Rastelement 68 formschlüssig mit einer dafür vorgesehenen Rastaufnahme 70 zusammenwirkt. Durch Zusammendrücken zweier Betätigungselemente 72 können das Rastelement 68 und die Rastaufnahme 70 voneinander getrennt werden, wie dies in der zweiten Darstellung von oben dargestellt ist. Entlang des Pfeiles 74 wird eine Zugkraft ausgeübt. In der dritten Darstellung von oben ist dargestellt, dass durch diese Zugkraft das Rastelement 68 verkippt wird, bis es in der nahezu senkrechten Position bezüglich der Zeichenebene steht, wie dies in der vierten Darstellung von oben gezeigt ist. Ein weiteres Entfernen der beiden Enden des Gegenlagerelementes 16 voneinander ist nicht möglich, da das Rastelement 68 in dieser Position nicht durch die Öse 76 hindurch passt. Dazu muss zunächst das Rastelement 68 verkippt werden, wie dies in der zweiten Darstellung von unten gezeigt ist. Erst in dieser Position passt es durch die Öse 76 und die beiden Elemente können voneinander getrennt werden, wenn Kraft entlang des Pfeiles 74 ausgeübt wird, wie dies in der untersten Darstellung dargestellt ist. Dadurch wird eine Sicherung in den Verschluss 66 eingeführt, durch den verhindert wird, das allein durch das Betätigen der Betätigungselemente 72 ein vollständiges Lösen und dadurch ein Zurückschnappen oder Zurückschlagen der einzelnen Enden des Gegenlagerelementes 16 hervorgerufen wird.

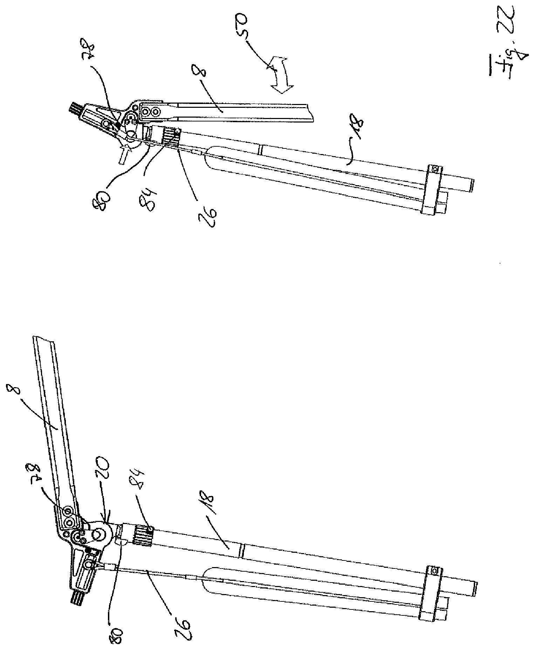

Figur 22 zeigt in der linken Darstellung wieder das Kraftübertragungselement 18, das Abstandselement 8 sowie den passiven Aktuator 26. Im Bereich des Gelenkes 20 ist zudem ein Anlageelement 78 vorhanden, das in der linken Darstellung von Figur 22 noch nicht am elastischen Element, das als passiver Aktuator wirkt, anliegt. Dies ist in der rechten Darstellung von Figur 22 anders. Man erkennt, dass das Anlageelement 78 an dem als passiven Aktuator wirkenden elastischen Element anliegt. Da sich das Anlageelement 78 vorzugsweise genau auf der Drehachse des Gelenkes 20 befindet, wird durch den Kontakt zwischen dem elastischen Element und dem Anlageelement 78 verhindert, dass das elastische Element eine Kraft auf das Armstützelement 6 aufgebracht wird, die ein Drehmoment auf das Armstützelement 6 hervorruft. Das elastische Element wird durch das anliegende Anlageelement 78 in zwei Teile geteilt, die zwar jeweils eine Kraft ausüben, jedoch kein Drehmoment erzeugen können, da die Kräfte radial auf die Drehachse des Gelenkes 20 gerichtet sind. Der Winkel zwischen dem Armstützelement 6 und dem Kraftübertragungselement 18, bei dem das Anlageelement 78 an dem elastischen Element anliegt, ist vorzugsweise einstellbar. Eine weitere Verschwenkung des Armstützelementes 6 im Uhrzeigersinn erfolgt dann, ohne dass durch das elastische Element, also den passiven Aktuator eine Kraft auf das Armstützelement 6 aufgebracht wird.

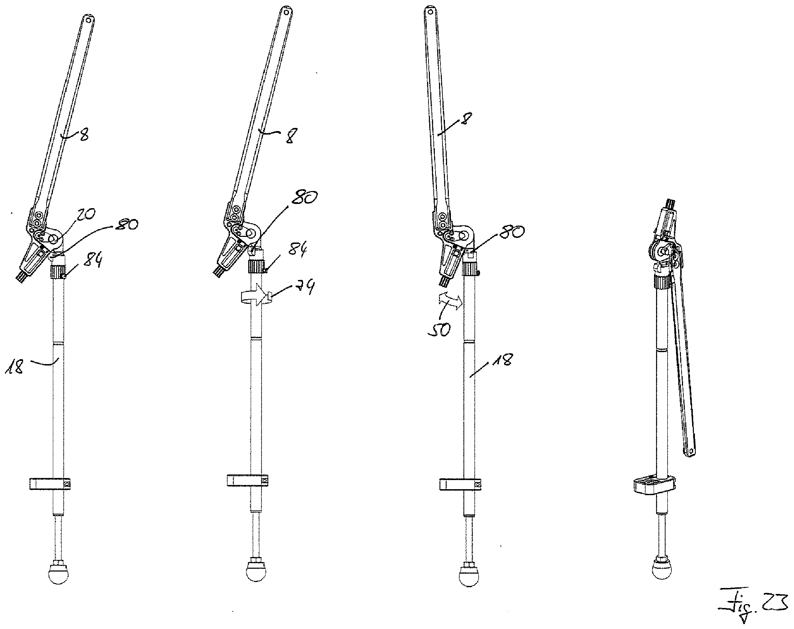

Figur 23 zeigt die Situation in der linken Darstellung. Der Anschlag 80 würde ein weites Verschwenken des Abstandselementes 8 relativ zum Kraftübertragungselement 18 um das Gelenk 20 herum verhindern. Soll die Vorrichtung beispielsweise verstaut oder der Vorsprung 80 in die passive Position gebracht werden, wird, wie in der zweiten Darstellung von links gezeigt, der Vorsprung 80 entlang des Pfeiles 74 außer Eingriff mit dem Abstandselement 8 gebracht. Nun kann wie in der zweiten Darstellung von rechts dargestellt entlang des Doppelpfeiles 50 in beide Richtungen das Abstandselement 8 völlig frei bezüglich des Kraftübertragungselementes 18 verschwenkt und so in die ganz rechts dargestellte Position gebracht werden.

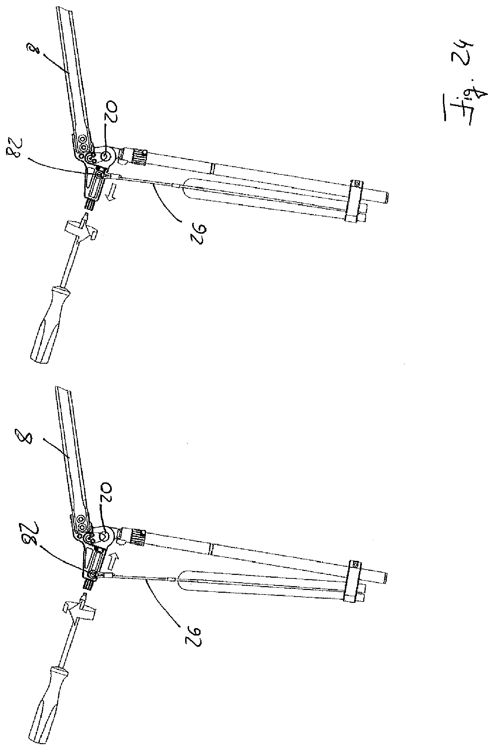

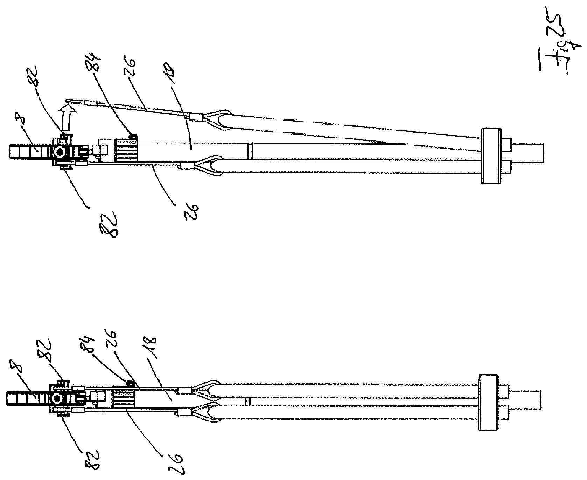

Figur 24 zeigt eine Möglichkeit, eine einstellbare Kraft, die durch den passiven Aktuator 26 aufgebracht wird, zu realisieren. Der passive Aktuator greift an einem Angriffspunkt 82, der bezüglich des Gelenkes 20 exzentrisch angeordnet ist, an dem Abstandselement 8 des Armstützelementes 6 an. Durch ein geeignetes Werkzeug, im gezeigten Ausführungsbeispiel ein Schraubendreher, kann über ein Formschlusselement die Position des Angriffspunktes 82 relativ zur Schwenkachse des Gelenkes 20 verstellt und so die Exzentrizität eingestellt und die durch den passiven Aktuator 26 aufgebrachte Kraft modifiziert werden. Eine alternativ oder zusätzlich dazu vorhandene Möglichkeit ist in Figur 25 dargestellt. Sie zeigt in beiden Darstellungen jeweils ein Kraftübertragungselement 18 sowie ein daran angeordnetes Abstandselement 8. Die Darstellung ist gegenüber der in Figur 24 gezeigten Darstellung um 90° gedreht. Es sind jeweils zwei passive Aktuatoren 26 vorhanden, die am gegenüberliegenden Angriffspunkt 82 an dem Abstandselement 8 angreifen. Soll nun die Kraft eingestellt werden, kann alternativ oder zusätzlich zu der in Figur 24 gezeigten Maßnahme auch einer oder beide der passiven Aktuatoren 26 entfernt und durch einen anderen ersetzt werden. Auch dadurch lässt sich die Kraft einstellen. Soll der passive Aktuator 26 mitgeführt werden, um beispielsweise zu einem späteren Zeitpunkt wieder verwendet zu werden, kann seine obere Befestigungsschlaufe an dem Vorsprung 84 angeordnet werden.

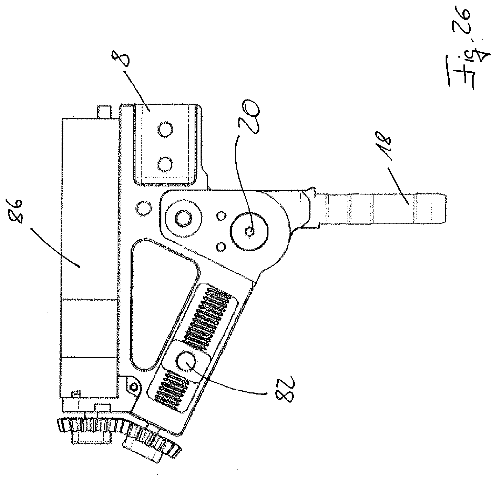

Figur 26 zeigt das Gelenk 20 mit dem Abstandselement 8 des Armstützelementes 6 und dem Kraftübertragungselement 18. Man erkennt, dass der Angriffspunkt 82 verschiebbar ausgebildet ist. Dazu verfügt die Vorrichtung über einen Motor 86, durch den die im linken Bereich der Figur 26 dargestellten Zahnräder antreibbar sind, wodurch der Angriffspunkt 82 bewegt wird. Durch den Motor kann das System in einen Zustand minimaler Kraft gebracht und so zumindest nahezu, vorzugsweise vollständig deaktiviert werden, was insbesondere beim An- und Ausziehen der Vorrichtung von Vorteil ist oder wenn die Unterstützung aus anderen Gründen nicht benötigt wird.

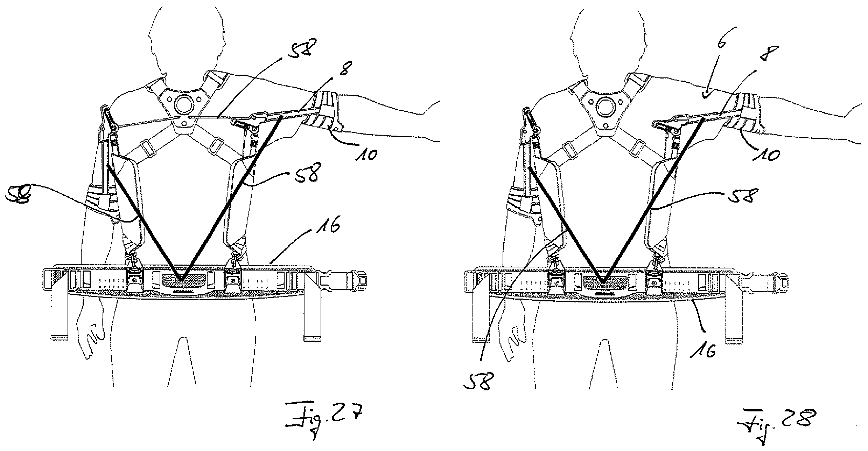

Figuren 27 und 28 entsprechen der Darstellung der Figur 18 mit dem Unterschied, dass zwei weitere Zugelemente 58 vorhanden sind, die nicht zwischen den Armstützen 6, sondern zwischen jeweils einer der Armstützen 6 und dem Gegenlagerelement 16 angeordnet sind. Auch durch diese werden die Armschalen 10 am Arm gehalten. Zudem sind die Elemente für die beiden Arme voneinander unabhängig, sodass eine Beeinflussung verhindert werden kann. In Figur 28 ist zudem das in Figur 18 gezeigte Zugelement 58 entfernt worden.

### Bezugszeichenliste

- 2: Benutzer
- 4: Arm
- 6: Armstützelement
- 8: Abstandselement
- 10: Armschale

- 12: Manschette
- 14: Gegenlager
- 16: Gegenlagerelement
- 17: Anlageelement
- 18: Kraftübertragungselement

- 20: Gelenk
- 22: Kugelgelenk
- 24: Einstellvorrichtung
- 26: passiver Aktuator
- 28: Hebelelement

- 30: Schultergurt
- 32: Verbindungselement
- 34: Schnalle
- 36: Gurt
- 38: Polsterung

- 40: Tasche
- 42: Exoskelett
- 44: Hüftelement
- 46: Bodenkontaktelement
- 48: Schwenkhebel
- 50: Doppelpfeil
- 52: Gegenspann
- 54: Verschlusselement
- 56: Umhüllung
- 58: Zugelement

- 60: Befestigungselement
- 62: Öse
- 64: Griffelement
- 66: Verschluss
- 68: Rastelement

- 70: Rastaufnahme
- 72: Betätigungselement
- 74: Pfeil
- 76: Öse
- 78: Anlageelement

- 80: Vorsprung
- 82: Angriffspunkt
- 84: Vorsprung
- 86: Motor

## Patentansprüche

1. Vorrichtung zum Unterstützen zweier Arme (4) eines Benutzers (2), wobei die Vorrichtung
- zwei Armstützelemente (6) mit je einer Armschale (10) zum Anlegen an jeweils einen Arm (4),
- wenigstens einen passiven Aktuator (26),
o der eingerichtet ist, eine Kraft auf wenigstens eines der Armstützelemente (6) aufzubringen, und
- wenigstens ein Gegenlager (14) für die aufzubringende Kraft aufweist, das
o wenigstens ein Gegenlagerelement (16) und
o wenigstens zwei Kraftübertragungselemente (18) aufweist, die eingerichtet sind, eine Gegenkraft von jeweils einem der Armstützelemente auf das Gegenlagerelement (16) zu übertragen,
wobei die Kraftübertragungselemente (18) relativ zu dem Gegenlagerelement (16) bewegbar, insbesondere um wenigstens eine Rotationsachse drehbar, an dem Gegenlagerelement (16) angeordnet sind,
**dadurch gekennzeichnet, dass** die Stärke der aufzubringenden Kraft einstellbar ist.

2. Vorrichtung nach Anspruch 1, **dadurch gekennzeichnet, dass** die Stärke der aufzubringenden Kraft durch eine veränderbare Vorspannung des passiven Aktuators (26) veränderbar ist.

3. Vorrichtung nach Anspruch 1 oder 2, **dadurch gekennzeichnet, dass** die Stärke der aufzubringenden Kraft durch eine einstellbare Exzentrizität der Kraftaufbringung auf das Armstützelement (6) veränderbar ist.

4. Vorrichtung nach einem der vorstehenden Ansprüche, **dadurch gekennzeichnet, dass** die Stärke der aufzubringenden Kraft durch eine Einstellung der entspannten Federlänge einstellbar ist.

5. Vorrichtung nach einem der vorstehenden Ansprüche, **dadurch gekennzeichnet, dass** die Einstellung der Stärke der aufzubringenden Kraft mechanisch oder mechatronisch, vorzugsweise motorgetrieben, vornehmbar ist.
